Europäisches Patentamt

European Patent Office

Office européen des brevets

(19)

(11) Publication number: **0 024 772**
**B1**

# (12) EUROPEAN PATENT SPECIFICATION

(45) Date of publication of patent specification: **13.06.84**

(51) Int. Cl.³: **A 61 B 5/02**

(21) Application number: **80200799.7**

(22) Date of filing: **25.08.80**

(54) **Apparatus for measuring the human being's blood pressure.**

(30) Priority: **28.08.79 CH 7785/79**

(43) Date of publication of application:
**11.03.81 Bulletin 81/10**

(45) Publication of the grant of the patent:
**13.06.84 Bulletin 84/24**

(84) Designated Contracting States:
**AT BE CH DE FR GB IT LI LU NL SE**

(56) References cited:
**CH - A - 557 671**
**FR - A - 1 307 275**
**FR - A - 1 334 572**
**FR - A - 2 052 617**
**US - A - 3 412 729**
**US - A - 3 482 565**
**US - A - 3 698 382**

**IBM Technical Disclosure Bulletin Vol. 6, No. 1,
June 1963, pages 85 and 86**

(73) Proprietor: **BATTELLE MEMORIAL INSTITUTE**
**7 route de Drize**
**CH-1227 Carouge/Genève (CH)**

(72) Inventor: **Prost, Jean-Louis**
**3, rue de l'Université**
**CH-1205 Geneva (CH)**

(74) Representative: **Dousse, Blasco et al,**
**7, route de Drize**
**CH-1227 Carouge/Genève (CH)**

Courier Press, Leamington Spa, England.

## Description

The present invention relates to an apparatus for measuring the human being's blood pressure.

The apparatus for measuring blood pressure that is most universally known comprises an inflatable cushion for compressing the arm until the blood circulation is interrupted, after which the pressure is progressively decreased and the reestablishment of the blood circulation is detected acoustically by means of the "Korotkoff noises" which are produced when the pressure decreases from the systolic pressure to the diastolic pressure. The "Korotkoff noises" are detected by means of a stethoscope placed between the inflatable cushion and the skin.

There was already proposed an automation of this apparatus for large public use by incorporating a microphone to the inflatable cushion and by connecting it to an electronic circuit for automatically determining the systolic and diastolic pressures.

The drawback of such apparatuses arises mainly from the fact that it is not easy to install oneself an inflatable cushion around the arm and that it is necessary to remove any clothing covering the arm. Although such constraints may be unimportant within the frame of the medical profession, they constitute a bar relative to a large public diffusion, especially when the pressure is taken by a person on oneself.

There was already proposed in French Patent No. 1.334.572 an apparatus for measuring blood pressure by the detection of light absorption variations through a portion of the earlap, consecutively to having put such portion of the earlap under variable pressure. Consequently, the application of this pressure drives the blood of this portion off the earlap and the detecting device will measure the volume of remaining blood. The measured value, of course, varies with the same frequency as the heart beat. For drawing a curve of the transparency variations, it is required to measure the maximal values and to calculate the curve from these values, after which the systolic and diastolic pressures are deduced from some specific values of this curve on the basis of particular relations existing between these values and the systolic and diastolic pressures.

The calculation of the curve of pressure variations requires the use of a relatively complex electronic circuit which results in a relatively expensive embodiment which is not suitable for making an apparatus intended for large public use. Moreover, the transparency measurement which is contemplated in this patent can only be measured on the earlap which is not an ideal measuring spot for an apparatus which could be put in anyone's hands and not reserved exclusively to doctors.

French Patent No. 2.052.617 and US Patent No. 3.412.729 concern oxymeters from which the blood pressure can be obtained indirectly. This measurement is based on the absorption of infrared light by tissues which varies in inverse proportion to the amount of blood, the latter varying in turn according to the blood pressure. the signal given by this measurement is alternating which makes the determination of the diastolic and systolic pressures relatively difficult to perform. Such apparatuses are generally intended for hospitals and are not suited for being sold outside the specialists field.

US Patent No. 3.698.382 concerns a device for driving the blood off the area underneath a portion of skin by means of a pressure application and for measuring the blood return rate in such area which is a function of the blood pressure. Such a device is however not adapted for measuring systolic and diastolic pressures.

As illustrated by IBM Technical Disclosure Bulletin Vol. 6, No. 1, June 1963, pages 85 and 86 as well as by US patent No. 3.482.565, works have already been carried out to attempt measuring the blood pressure from a finger. In such a case, the blood stream can be detected optically by means of a photoplethysmograph which measure the light through the skin which varies as a function of the heart beat. Consequently, when the blood circulation is stopped, the signal from the photoplethysmograph is essentially constant and it is alternatively variable when the circulation is reestablished.

These last reference processes enable to essentially measure the systolic pressure, with extremely questionable reliability. With these processes, the measure of diastolic pressure has no practical significance since large variations occur from one to the other measurement.

The difficulties result from several factors, namely the drop of load between the arm and the fingers where the arteries have a much lower diameter. If the measured pressure is correct, it is however different from the value obtained on the arm which constitutes a universal standard. Moreover, if this pressure is different from that measured on the arm, it can vary under the influence of other factors so that it is even difficult to set up comparisons between several measurements on the same person. This difference between several measurements on the same person essentially results from the vaso-constrictive automatic control of the quantity of blood that circulates in the terminal members (hands and feet) as a function of the needs in energy of other parts of the body. Thus, the amount of blood in the fingers is smaller during digestion or when the weather is cold, which is an additional factor which may alter the pressure drop between the arm and the fingers and also in the case of two measurements done on a finger when the time between the two is significant. Thus, not only is the finger measurement not comparable to the arm measurement, but two successive finger measurements done under different conditions are no more comparable most often.

The aim of the present invention is to remedy, at least in part, the above-mentioned drawbacks.

Hence, the present invention has for object an apparatus for measuring the human being's blood pressure according to the claim.

The essential advantage of this apparatus results from the fact that it enables directly measuring a continuous evolution of the amount of pressure thanks to the fact that the pressure detector is applied against the measuring area with a determined pressure sufficiently weak, however, not to interfere with the pressure exerted upstream from this measuring area. This occurence is an unexpected phenomenon which enables a considerable simplification of the electronic circuit for processing the measured signals.

The annexed drawing schematically illustrates, as an Example one embodiment of the apparatus according to the present invention.

Fig. 1 is a schematic view of a measuring element,

Fig. 2 is a block-diagram of this embodiment,

Fig. 3 shows a diagram of the recorded signals.

The optical-electronic observation of the transparency of superficial tissues enables to detect an alternating signal the frequency of which corresponds to that of the heart beat rhythm. This measure can be done with a photoplethysmograph which comprises a cell 1 with a transparent detector face divided into two parts by a partition. One of the parts has a light source, for instance infra-red, and the other has a photo-electric detector. This cell can be, for instance, a cell for detecting objects by reflexion "OPTRON" (Trade Mark), Type OPB-730. This cell is enclosed in a cap 2 having a recess 3 for accomodating the tip of the finger to be measured. This cap is provided inside with a resiliently compressible material such as a plastic foam 4 for ensuring a contact of the cell with the finger and to absorb, to a certain extent, the variations of finger size. In connection with the cap 2, an organ 5 for blocking the blood flow is placed upstream in regard to the arterial circulation.

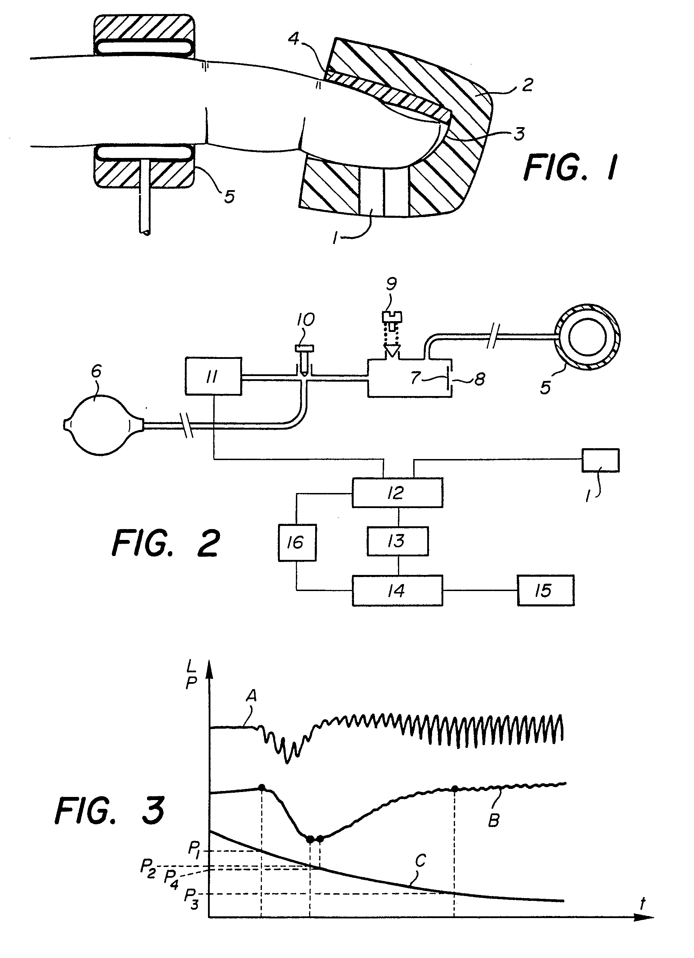

Systematic transparency measurements, carried out downstream from a portion of a finger the blood flow of which has been stopped by a blocking device 5, have shown that, when the blood circulation is restored, the average transparency curve which can also be called the continuous transparency evolution drops suddenly to reach a minimum value after which it progressively rises again to finally get stabilized to a practically constant level. This curve is illustrated on the diagram of Fig. 3 by reference B. On this diagram have been drawn, the curves A and B corresponding to the light intensity L vs. time t and curve C corresponding to the pressure P vs. time t. It must be noted that the curve B, which is the same for any individual, can only be measured if the contact pressure between the photoplethysmograph is between well defined limits ranging from 5000 Pa to 15000 Pa. The experiments undertaken have been done with a pressure of about 10000 Pa. There was noted, indeed, that if the pressure is too low or too large one still measures an alternating signal as a function of the cardiac rhythm, but not the curve B. Now, the usefulness of this curve is that it is a characteristic of the blood circulation consecutive to the release of the blocking pressure. Moreover, this curve has some inflexion points which constitute guidemarks which are particularly well suited for electronic processing. It is however not possible to achieve a direct relationship between this curve B and the "Korotkoff noises". Thus, this curve does not enable to directly infer the systolic and diastolic pressures, especially if one desires that such pressures be comparable to the corresponding pressures measured on the arm and be relatively independent of the physiological conditions under which the measurements are undertaken.

Consequently, the measured pressures must be processed for enabling to evaluate the systolic and diastolic pressures.

This treatment implies a standardization of the apparatus and of its operating processes. For achieving such standardization, there were undertaken a series of measurements on a sample number of people, systematically correlating the measurement done on the arm and that done on the finger. Thanks to these measures, it was possible to statistically establish a correlation between the pressures measured on the arm, and considered as being reference pressures, and the pressures measured as a function of the various inflexion points of the curve B. These characteristic pressure values of these inflexion points form a group of three, such being defined $P_1$, $P_2$ and $P_3$. $P_1$ corresponds to the characteristic pressure of the first bending point of curve B. This first inflexion point coincides with the returning of the blood circulation and theoretically corresponds to the systolic pressure in the finger but for which there is a bad reproducibility for the reasons previously explained and which can be appreciably different from the reference pressure. $P_2$ corresponds to the pressure characteristic at the place where the curve B goes through a minimum and $P_3$ corresponds to the pressure characteristic of the spot where the curve stops rising after the minimum point for staying constant. These pressures are measured by correlating the curve B and the curve C that corresponds to the pressure drop in the blocking air cushion of the finger.

The sampling thus achieved enabled to obtained a good evaluation, in the average at $\pm$ 5%, of the reference systolic pressure $P_s$ through the following equation:

$$P_s \cong \frac{P_1 + P_2 + P_3}{2}$$

This calibration corresponds to an approximation based on this sample number of people and is statistically valid for a larger scale of individuals. However, this calibration does not take into account some individuals with pathological disorders of the blood stream. It is then possible to contemplate an individual calibration of the process. Although, this possibility of individual calibration does exist, it should not be forgotten that the process according to the invention has not for an object to replace the existing sphygomanometers used by medical practitioners but to offer a simple means that can be easily undertaken for enabling anyone to measure its pressure and for giving a result comparable to the pressure measured on the arm.

By the same statistical measurements, it was possible to define a calibration which enabled to evaluate the diastolic pressure relative to the pressure measured on the arm. If one refers again to the diagram of Fig. 3, the curve A corresponds to the alternating variation of the transparency of the finger, the average level of which is given by curve B. This alternating variation is a characteristic of the heart rhythm. There was noticed that one statistically obtains a good approximation of the reference diastolic pressure by measuring a pressure $P_4$ that is the characteristic pressure of the heart beat following the minimum bending point of curve B. Hence, it is sufficient to identify on curve A, which gives the transparency variations as a function of the heart rhythm, the beat that follows the minimum inflexion point on curve B and to check at this moment the pressure value on curve C to obtain the evaluation of the diastolic reference pressure.

The block diagram of Fig. 2 shows the organ 5 for blocking the blood flow including an inflatable cushion connected to a pressure source made of a pumping element 6 and a check valve 7 combined with an inlet 8. A safety valve 9 enables the air to escape when the pressure exceeds a given limit. A controlled release valve 10 is for progressively reducing the pressure in the blocking organ 5 for restoring the blood flow. This air circuit for applying pressure is connected to a pressure transducer 11 the output of which is connected to a signal shaper and amplifying circuit 12.

The circuit 12 is further connected to the output of the optical-electronic detector cell 1 of the photoplethysmograph so that the two informations are processed parallelwise. The output of this circuit 12 is connected to a digital-analog converter 13, the output of which is connected to a computing unit 14 bound to a display device 15. The computing unit 14 is that unit in which the different pressures measured are processed for obtaining an estimation of the reference systolic and diastolic pressures on the basis of the finger measured pressures. The electronic circuit is powered by a voltage source 16.

This apparatus has been tested to a rather important scale on several tens of people and at different times. Each trial was preceded by a measure of the reference pressure done on the arm. The results obtained are generally near to the reference measured values. In all cases, it can be noticed that the apparatus, disclosed enables improving the accuracy of the evaluation of the systolic pressure $P_s$ relatively to the pressure $P_1$ which might be considered the systolic pressure since it corresponds to the characteristic pressure at the time the blood flows again. This does not mean that the pressure $P_1$ is wrong, but only it is not comparable to the reference systolic pressure universally accepted, whereas the measuring process enables to come nearer this reference pressure $P_s$ and to also have a more reproducible value, thus less dependent from the physiological factors likely to vitiate the measurement. The values measured for the diastolic pressure are equally interesting in the sense that it was nearly impossible before to optically measure the acoustical equivalent of the vanishing of the "Korotkoff noises". This determination, done according to the given method in taking the average transparency curve B as a landmark, is a startling and also reproducible result.

The apparatus statistically calibrated as described is simple, easy to operate, practical and offering to the large public a control means compatible with the sphygmomanometers commonly used in the medical professions.

**Claim**

Apparatus for measuring the human being's blood pressure comprising:

a cuff (5) provided with an air-inflatable cushion applicable to a distal portion of a human being for exerting an astringent pressure temporarily cutting of the blood flow,

operating means (6 to 10) connected with said cushion for inflating, and controllably deflating same,

transducer means (11) coupled with said operating means (6 to 10) for converting measured values of said astringent pressure into first electrical signals,

a detector (1) including a light source and a photosensor for measuring the change of transparency of a skin area,

adapting means (2, 4) for applying said detector (1) on a skin area downstream with respect to the cuff (5),

the photosensor being connected to a circuit means (12) for converting the output signal of the photosensor into a second electrical signal representative of the transparency of the skin area,

said light source and said photosensor are disposed side by side, that,

adapting means (2, 4) comprises elements for applying said detector (1) with a contact pressure ranging between 500 and 15000 Pa so that said second electrical signal is representative of the average transparency (curve B) of said skin area,

that said transducer means (11) is connected to said circuit means (12) for shaping and amplifying said first signals,

and that a computing means (14) connected to said circuit means (12) is provided for determining blood pressure values characteristic of the average transparency of the skin area.

## Revendication

Appareil pour mesurer la pression sanguine d'un être humain comprenant:

une machette (5) munie d'un coussin gonflable applicable à une partie d'extémité du corps humain pour exercer une pression astringente arrêtant temporairement l'écoulement du sang,

des moyens d'actionnment (6 à 10) reliés à ce coussin pour le gonfler et le dégonfler de façon réglable,

un organe transducteur (11) couplé auxdits moyens d'actionnement (6 à 10) pour convertir les valeurs mesurées de ladite pression astringente en premiers signaux électriques,

un détecteur (1) comprenant une source de lumière et un photo-détecteur pour mesurer le changement de transparence d'une zone de peau,

des moyens d'adaptation (2, 4) pour appliquer ce détecteur (1) sur une zone de peau en aval par rapport à la manchette (5),

le photo-détecteur étant relié à un circuit (12) pour convertir le signal de sortie du photo-détecteur en un second signal électrique représentatif de la transparence de la zone de peau, caractérisé par le fait que

ladite source de lumière et ledit photo-détecteur sont disposés côte à côte,

que lesdits moyens d'adaptation (2, 4) comportent des éléments pour appliquer ledit détecteur (1) avec une pression de contact comprise entre 5000 et 15000 Pa de sorte que ledit second signal électrique est représentatif d'une transparence moyenne (courbe B) de cette zone de peau,

que ledit organe transducteur (11) est connecté audit circuit (12) pour mettre en forme et amplifier lesdits premiers signaux et

qu'un organe de calcul (14) relié audit circuit (12) est agencé pour déterminer les valeurs de pression du sang caractéristiques de la transparence moyenne de la zone de peau.

## Pantentanspruch

Gerät zum Messen des Blutdruckes eines Menschen, aufweisend:

eine Manschette (5), die mit einem aufblasbaren Kissen versehen ist, das an einem körperfernen Abschnitt eines Menschen angeordnet werden kann, um einem susammenpressenden Druck auszuüben, der zeitweilig den Blutfluß unterbricht,

Betätigungsanordnungen (6 bis 10), die mit dem Kissen verbunden sind, um dieses aufzublasen und gesteuert Luft abzulassen,

mit den Betätigungsanordnungen (6 bis 10) gekoppelte Wandleranordnungen (11), um gemessene Werte des zusammenpressenden Druckes in erste elektrische Signale umzuwandeln,

einen Detektor (1) mit einer Lichtquelle und einem Photosensor zum Messen de Durchlässigkeitsänderung eines Hautberreiches,

Passungsanordnungen (2, 4) zum Anordnen des Detektor (1) an einem Hautbereich unterhalb der Manschette (5), wobei der Photosensor mit einem Kreis (12) zur Umwandlung des Ausgangssignales des Photosensors in ein zweites elektrisches Signal verbunden ist, das für die Durchlässigkeit des Hautbereiches kennzeichnend ist; dadurch gekennzeichnet, daß die Lichtquelle und der Photosensor nebeneineander angeordnet sind,

daß die Passungsanordnungen (2, 4) Elemente aufweisen, um den Detektor (1) mit einem Kontaktdruck im Bereich zwischen 5000 und 15000 Pa aufzubringen,

sodaß das zweite elektrische Signal für die mittlere Durchlässigkeit (Kurve B) des Hautbereiches kennzeichnend ist,

daß die Wandleranordnungen (11) zur Formung und Verstärkung des ersten Signals mit dem Kreis (12) verbunden sind und

daß ein mit dem Kreis (12) verbundener Rechner (14) vorgesehen ist, um für die mittlere Durchlässigkeit des Hautbereiches charakteristische Blutdruckwerte zu bestimmen.

FIG. 1

FIG. 2

FIG. 3